# FASCICULE DE BREVET EUROPEEN

(11) **EP 0 403 395 B1**
(45) Date de publication et mention de la délivrance du brevet: **03.04.1996**
(21) Numéro de dépôt: 90401684.7
(22) Date de dépôt: 15.06.1990
(51) Int. Cl.: C08F 220/56

(54) **Procédé de préparation de poly beta-alanine réticulée en une seule étape,à partir d'acrylamide et d'un composé polyfonctionnel copolymérisable**
Verfahren zur Herstellung von vernetztem Polybeta-alanin in einem einzigen Schritt aus Acrylamid und einer polyfunktionalen copolymerisierbaren Verbindung
Process for the one-step preparation of cross-linked poly beta-alanine from acrylamide and a polyfunctional compound copolymerisable therewith

(30) Priorité: 15.06.1989 FR 8907946
(43) Date de publication de la demande: 19.12.1990
(73) Titulaire: L'OREAL, F-75008 Paris (FR)
(72) Inventeur: Lapoiriere, Claude, F-94170 Le Perreux (FR); Mahieu, Claude, F-75017 Paris (FR); Papantoniou, Christos, F-95160 Montmorency (FR)
(74) Mandataire: Stalla-Bourdillon, Bernard

(56) Documents cités:
- FR-A- 2 530 250

## Description

La présente invention a pour objet un nouveau procédé de préparation de microsphères de poly β-alanine réticulée ainsi que les microsphères ainsi obtenues, celles-ci pouvant être imprégnées d'une substance active, pharmaceutique ou cosmétique.

Il a déjà été proposé une poly β-alanine réticulée qui a été décrite notamment dans le brevet français n°83.11609 (2.530.250) ainsi d'ailleurs que son procédé de préparation.

Selon le procédé de ce brevet, cette poly β-alanine réticulée est obtenue en deux étapes, la première consistant à polymériser l'acrylamide en vue d'obtenir la poly β-alanine soluble dans l'eau et la seconde consistant à réticuler, en suspension dans un solvant organique, une solution aqueuse de poly β-alanine à l'aide d'un agent réticulant notamment un dialdéhyde tel que le glutaraldéhyde.

Les sphères de poly β-alanine réticulée obtenues selon ce procédé sont alors soumises à une opération de tamisage en vue d'obtenir des microsphères possédant une certaine homogénéité de taille.

Selon le procédé de ce brevet, les microsphères sont formées lors de l'étape de réticulation et leur taille dépend essentiellement de la nature et de la quantité de l'agent de suspension utilisé.

Ce procédé qui nécessite deux étapes ainsi qu'une opération ultérieure de tamisage est particulièrement lourd à mettre en oeuvre et de ce fait influe de façon non négligeable sur le prix de revient de la poly β-alanine réticulée.

Après diverses études, il a été constaté que l'on pouvait obtenir une poly β-alanine réticulée présentant d'excellentes propriétés, et ayant par ailleurs une faible dispersité de taille des particules, en utilisant un procédé ne comportant qu'une seule étape.

On a en effet remarqué qu'en utilisant comme agents réticulants des composés polyfonctionnels copolymérisables avec l'acrylamide, il était possible d'obtenir dans certaines conditions de polymérisation des microsphères de poly β-alanine réticulée tout à fait comparables quant à leurs propriétés à celles obtenues par le procédé du brevet français n°83.11609 utilisant comme agent réticulant un dialdéhyde tel que le glutaraldéhyde.

Ce procédé est particulièrement intéressant d'un point de vue industriel non seulement par le fait qu'il permet d'obtenir une poly β-alanine réticulée en une seule étape mais également par le fait que l'on obtient des microsphères possédant une certaine homogénéité de taille ce qui permet d'éviter l'opération de tamisage indispensable dans le cadre du brevet français n°83.11609.

Le procédé selon l'invention est essentiellement caractérisé par le fait qu'il consiste à polymériser de l'acrylamide avec un composé polyfonctionnel copolymérisable avec l'acrylamide, en tant qu'agent réticulant, la polymérisation étant conduite dans un solvant organique ou un mélange de tels solvants en présence d'un initiateur anionique de polymérisation et d'un agent de suspension.

Les composés polyfonctionnels copolymérisables avec l'acrylamide, utilisés comme agent réticulant selon le procédé de l'invention, sont de préférence des composés polyfonctionnels insaturés ayant des fonctions acrylamides, méthacrylamides, esters acryliques, vinyliques ou allyliques.

Selon un mode de réalisation préféré de l'invention, ces composés polyfonctionnels insaturés sont de préférence porteurs de fonctions acrylamides ou méthacrylamides.

Parmi ceux-ci on peut notamment mentionner les suivants:
1,2-dihydroxyéthylène bis-acrylamide, N-N′-(bis-acrylyl) cystéamine, diméthylène bis-acrylamide, hexaméthylène bis-acrylamide, octaméthylène bis-acrylamide, dodécaméthylène bis-acrylamide, m-xylylène bis-acrylamide, butylidène bis-acrylamide, benzylidène bis-acrylamide, acide diacrylamido acétique, xylylidène tétra-acrylamide, N,N′-bis-(acrylyl) pipérazine et p-benzylidène camphre N,N′-acrylyl diaminométhane.

Le p-benzylidène camphre N,N′-acrylyl diaminométhane est décrit dans le brevet français n°85.12959 (2.586.693).

Les études qui ont été réalisées à l'aide de divers types de tels composés polyfonctionnels insaturés ont permis de mettre en évidence qu'il était préférable d'utiliser des composés bi-fonctionnels.

Bien qu'il ait été fait référence ci-dessus à certains composés préférés, il va de soi que l'invention n'en est pas pour autant limitée à leur seul usage et que d'autres composés polyfonctionnels copolymérisables avec l'acrylamide peuvent être utilisés comportant notamment d'autres fonctions telles que par exemple des fonctions époxy.

Le solvant organique du procédé selon l'invention peut être du tertio-butanol ou du toluène mais de préférence un mélange de tertio-butanol et de toluène dans des rapports compris entre 1:24 et 10:1 et de préférence entre 1:6 et 6:1.

On a constaté à cet égard de façon tout à fait surprenante qu'en utilisant un tel mélange solvant dans les rapports mentionnés ci-dessus, il était possible d'obtenir des microsphères de poly β-alanine ayant une très faible dispersité de taille.

En ce qui concerne la taille même des microsphères, celle-ci dépend essentiellement du rapport entre le tertiobutanol et le toluène mais également du rapport du mélange solvant à l'acrylamide et de la vitesse d'agitation.

De façon générale, plus on augmente la proportion de tertiobutanol par rapport au toluène, plus le diamètre des microsphères diminue.

L'initiateur anionique de polymérisation est de préférence du tertiobutylate de sodium ou de potassium utilisé en une proportion d'environ 0,1 à environ 2 moles % par rapport à l'acrylamide.

L'agent de suspension du procédé selon l'invention est de préférence le copolymère octadécène-anhydride maléïque et plus particulièrement le copolymère vendu sous la dénomination "PA 18" par la Société GULF.

La quantité d'agent de suspension est généralement comprise entre 0,1 et 2% en poids par rapport à l'acrylamide.

La température de polymérisation est de l'ordre de 60°C à environ 100°C mais de préférence d'environ 80°C.

Les microsphères de poly β-alanine réticulée telles qu'obtenues par le procédé selon l'invention trouvent tout particulièrement une application dans le domaine thérapeutique, celles-ci pouvant être appliquées sur des plaies infectées ou non et agir en tant qu'agent d'absorption facilitant les processus naturels de cicatrisation.

Les microsphères de poly β-alanine réticulée peuvent également servir de support pour certaines substances actives.

En vue d'introduire les substances actives dans les microsphères de poly β-alanine réticulée, on dissout le produit actif dans un solvant ou un mélange de solvants ayant une affinité suffisante vis-à-vis de la poly β-alanine réticulée. Parmi les solvants appropriés on peut notamment citer l'eau, le glycérol, l'éthanol, le diéthylèneglycol, le propylèneglycol et leurs mélanges ou les mélanges d'eau et de solvants organiques miscibles à l'eau comme par exemple l'acétone.

Lorsque l'on utilise un solvant pour obtenir des microsphères de poly β-alanine chargées en un produit actif, on peut utiliser les microsphères telles quelles ou éventuellement après élimination du solvant utilisé pour leur imprégnation. Ce solvant peut avoir subsisté comme solvant du produit actif et/ou comme agent de gonflement des microsphères elles-mêmes lorsque celles-ci sont susceptibles de gonfler dans le solvant. Lorsque les microsphères de poly β-alanine réticulée sont utilisées après élimination du solvant, le produit actif reste néanmoins, après séchage piégé dans (ou/sur) les microsphères. Le gonflement de la poly β-alanine réticulée, par un solvant, conduit à des microsphères sous forme de gel, sous réserve que la quantité de solvant ne dépasse pas certaines limites qui sont différentes selon la nature exacte des microsphères de poly β-alanine réticulée.

Les microsphères chargées d'au moins une substance active,séchées ou non, peuvent alors être mélangées à un véhicule pharmaceutique ou cosmétique.

Parmi les substances actives on peut mentionner les suivantes:
Humectants, antisolaires, anti-inflammatoires, anti-fongiques, antibiotiques, antiacnéïques, anti-purigineux, agents de ramollissement des ongles, agents de raffermissement de la peau, agents de traitement des cheveux, astringents, vitamines, huiles essentielles, collagène, etc.

On va maintenant donner à titre d'illustration et sans aucun caractère limitatif plusieurs exemples du procédé de préparation de la poly β-alanine réticulée selon l'invention et des exemples d'imprégnation des microsphères ainsi obtenues à l'aide de diverses substances actives.

### EXEMPLE 1

Dans un reacteur de 2 litres, muni d'une agitation mécanique à 500t/mn, d'un réfrigérant, d'une arrivée d'azote et d'un moyen de régulation de la température, on introduit 300g de toluène, 147g de tertiobutanol et 0,5g de copolymère octadécène-anhydride maléïque (vendu sous la dénomination de "PA-18" par la Société GULF). Après chauffage de ce mélange à 70°C on ajoute 45g d'acrylamide et 5g de butylidène bis-acrylamide dans 50g de toluène. On porte alors la température à 80°C pendant 15mn environ. On ajoute ensuite en 10mn une solution de 1,12g de tertiobutylate de potassium dans 20g de tertiobutanol, celle-ci ayant été obtenue en portant le mélange au reflux pendant 2 heures.

Après agitation pendant 6 heures à 80°C, on laisse revenir à température ambiante puis on filtre les microsphères de poly β-alanine réticulée. Après lavage à l'éthanol et à l'eau, les microsphères de poly β-alanine réticulée sont ensuite séchées par lyophilisation (rendement: 40%). Les microsphères de poly β-alanine réticulée se présentent sous forme d'une poudre blanche, dont le diamètre moyen des microsphères est en moyenne de 6µm déterminé par la technique d'analyse d'images sur un appareil "QUANTIMET 900" de la Société CAMBRIDGE INSTRUMENTS CO.

### EXEMPLE 2

Dans un réacteur de 2 litres, muni d'une agitation mécanique à 500t/mn, d'un réfrigérant, d'une arrivée d'azote et d'un moyen. de régulation de la température, on introduit 300g de toluène, 147g de tertiobutanol et 0,5g de copolymère octadécène-anhydride maléïque (vendu sous la dénomination de "PA-18" par la Société GULF). Après chauffage de ce mélange à 70°C on ajoute 48,75g d'acrylamide et 1,25g de diméthylène bis-acrylamide dans 50g de toluène. On porte alors la température à 80°C pendant 15mn environ. On ajoute ensuite en 10mn une solution de 1,12g de tertiobutylate de potassium dans 20g de tertiobutanol, celle-ci ayant été obtenue en portant le mélange au reflux pendant 2 heures.

Après agitation pendant 5 heures à 80°C, on laisse revenir à température ambiante puis on filtre les microsphères de poly β-alanine réticulée. Après lavage à l'éthanol et à l'eau, les microsphères de poly β-alanine réticulée sont ensuite séchées par lyophilisation (poids obtenu: 40,5g). Les microsphères de poly β-alanine réticulée se présentent sous forme d'une poudre blanche, dont le diamètre moyen des microsphères est de 7µm déterminé par la technique d'analyse d'images sur un appareil "QUANTIMET 900" de la Société CAMBRIDGE INSTRUMENTS CO.

### EXEMPLES D'APPLICATION

### EXEMPLE A

Dans une solution de 44,5g de peroxyde de benzoyle (75% en poids) dans un mélange de 1125g d'acétone et de 375cm³ d'eau, on met en suspension 50g de microsphères de poly β-alanine réticulée obtenues selon l'Exemple 1 ci-dessus.

La suspension est ensuite concentrée à l'évaporateur rotatif, sous pression réduite et à une température inférieure à 35°C, jusqu'à un poids total de 262g de suspension.

La teneur de la suspension obtenue en peroxyde de benzoyle est de 9,1% en poids.

### EXEMPLE B

Dans une solution de 2g de nicotinate de benzyle dans un mélange de 40cm³ d'eau et de 40g d'éthanol, on met en suspension 10g de microsphères de poly β-alanine réticulée obtenues selon l'Exemple 2 ci-dessus. La suspension est maintenue sous agitation pendant 2 heures, puis l'éthanol est éliminé à l'évaporateur rotatif à une température inférieure à 35°C. Les microsphères sont alors séchées par lyophilisation.

### EXEMPLE C

On dissout 15mg de butyl-hydroxy-toluène (antioxydant) dans 30g de 1,2-propylèneglycol à une température de 30°C. Après refroidissement à température ambiante, on ajoute au mélange obtenu 24mg d'acide rétinoïque sous argon et à l'abri de la lumière. La solution obtenue est filtrée à l'aide de filtres "Millipore" 0,2µm. On met alors en suspension dans cette solution, à l'abri de la lumière et sous courant d'argon, 5g de microsphères de poly β-alanine réticulée obtenues selon l'Exemple 2 ci-dessus.

On mélange à l'aide d'une spatule et après 2 heures d'absorption on obtient une poudre jaune. L'acide rétinoïque est dosé par spectrophoto- métrie (λ = 358,8nm) après désorption du principe actif dans du diméthylsulfoxide.

| | |
|---|---|
| Concentration théorique : | 0,16% |
| Concentration calculée: | 0,157% |

### EXEMPLE D

A une solution de 3g d'huiles essentielles du parfum "LOULOU" de la Société CACHAREL dilués par 9g de propylèneglycol à 50°C, on introduit 4g de microsphères de poly β-alanine réticulée obtenues selon l'Exemple 1 ci-dessus tout en malaxant le mélange. On obtient un gel poudreux et parfumé qui est conditionné dans des flacons.

### EXEMPLE E

On dissout 20g de benzylidène-3 dl-camphre dans un mélange composé de 230g d'éthanol et de 110cm³ d'eau préalablement chauffé à 40°C. La solution est alors filtrée puis on y introduit 20g de microsphères de poly β-alanine réticulée obtenues selon l'Exemple 2 ci-dessus. On élimine alors l'éthanol sous pression réduite à 40°C puis l'eau par lyophilisation.

### EXEMPLE F

2g de Minoxidil sont dissous à 30°C dans un mélange de 75g d'éthanol et 75cm³ d'eau. A cette solution on introduit alors 8g de microsphères de poly β-alanine réticulée obtenues selon l'Exemple 2 ci-dessus. Après agitation du mélange pendant 1 heure à l'évaporateur rotatif, on évapore le solvant jusqu'à l'obtention d'un gel. Le gel est ensuite congelé sous agitation puis lyophilisé.

Concentration calculée : 11,6% (par dosage UV à 230nm après mise en suspension dans de l'éthanol).

## Revendications

1. Procédé de préparation de poly-β-alanine réticulée sous forme de microsphères ayant une faible dispersité de taille, caractérisé par le fait qu'il consiste à polymériser de l'acrylamide avec, en tant qu'agent réticulant, un composé polyfonctionnel copolymérisable avec l'acrylamide en une proportion suffisante par rapport à l'acrylamide pour réaliser la réticulation, ledit procédé étant réalisé dans un solvant organique choisi dans le groupe constitué par le tertiobutanol, le toluène et les mélanges tertio-butanol/toluène dans des rapports compris entre 1:24 et 10:1 et de préférence entre 1:6 et 6:1, en présence d'un initiateur anionique de polymérisation et d'un agent de suspension.

2. Procédé selon la revendication 1, caractérisé par le fait que l'initiateur anionique de polymérisation est le tertio-butylate de sodium ou de potassium utilisé en une proportion de 0,1 à 2 moles % par rapport à l'acrylamide.

3. Procédé selon la revendication 1, caractérisé par le fait que l'agent de suspension est le copolymère octadécène-anhydride maléïque.

4. Procédé selon la revendication 1, caractérisé par le fait que le composé polyfonctionnel copolymérisable avec l'acrylamide est porteur de fonctions acrylamides ou méthacrylamides.

5. Procédé selon l'une quelconque des revendications 1 et 4, caractérisé par le fait que le composé polyfonctionnel copolymérisable avec l'acrylamide est choisi parmi les composés suivants :
1,2-dihydroxyéthylène bis-acrylamide, N-N'-(bis-acrylyl) cystéamine, diméthylène bis-acrylamide, hexaméthylène bis-acrylamide, octaméthylène bis-acrylamide, dodécaméthylène bis-acrylamide, m-xylylène bis-acrylamide, butylidène bis-acrylamide, benzylidène bis-acrylamide, acide diacrylamido acétique, xylylidène tétra-acrylamide, N,N'-bis-(acrylyl) pipérazine et p-benzylidène camphre N,N'-acrylyl diaminométhane.

6. Microsphères de poly-β-alanine réticulée telles qu'obtenues selon le procédé des revendications 1 à 5.

## Claims

1. Process for the preparation of cross-linked poly-β-alanine in the form of microspheres having a low size dispersity, characterized in that it consists in polymerizing acrylamide with, as cross-linking agent, a polyfunctional compound copolymerizable with acrylamide in a sufficient proportion relative to the acrylamide in order to carry out the cross-linking, the said process being carried out in an organic solvent chosen from the group consisting of tert-butanol, toluene and tert-butanol/toluene mixtures in ratios of between 1:24 and 10:1 and preferably of between 1:6 and 6:1, in the presence of an anionic polymerization initiator and a suspending agent.

2. Process according to Claim 1, characterized in that the anionic polymerization initiator is sodium or potassium tert-butoxide used in a proportion of 0.1 to 2 mol % relative to the acrylamide.

3. Process according to Claim 1, characterized in that the suspending agent is the octadecene-maleic anhydride copolymer

4. Process according to Claim 1, characterized in that the polyfunctional compound copolymerizable with acrylamide carries acrylamide or methacrylamide functional groups.

5. Process according to either of Claims 1 and 4, characterized in that the polyfunctional compound copolymerizable with acrylamide is chosen from the following compounds:
1,2-dihydroxyethylenebisacrylamide, N,N'-(bis-acrylyl)cysteamine, dimethylenebisacrylamide, hexamethylenebisacrylamide, octamethylenebisacrylamide, dodecamethylenebisacrylamide, m-xylylenebisacrylamide, butylidenebisacrylamide, benzylidenebisacrylamide, diacrylamidoacetic acid, xylylidenetetraacrylamide, N,N'-bis(acrylyl)piperazine and p-benzylidenecamphor N,N'-acrylyldiaminomethane.

6. Cross-linked poly-β-alanine microspheres as obtained according to the process of Claims 1 to 5.

## Patentansprüche

1. Verfahren zur Herstellung von vernetztem Poly-β-alanin in Form von Mikrokügelchen mit einer geringen Größenstreuung, dadurch gekennzeichnet, daß das Verfahren aus der Polymerisation von Acrylamid mit einer mit Acrylamid copolymerisierbaren polyfunktionellen Verbindung als Vernetzungsmittel in einem Mengenverhältnis besteht, das in bezug auf das Acrylamid zum Durchführen der Vernetzung ausreichend ist, wobei das Verfahren in einem organischen Lösungsmittel durchgeführt wird, das aus der aus tert-Butanol, Toluol und Gemischen aus tert-Butanol und Toluol bestehenden Gruppe ausgewählt ist, wobei deren Mengenverhältnisse zwischen 1 : 24 und 10 : 1, vorzugsweise zwischen 1 : 6 und 6 : 1 betragen, und das Verfahren in Anwesenheit eines anionischen Polymerisationsstarters und eines Suspendiermittels durchgeführt wird.

2. Verfahren nach Anspruch 1, dadurch gekennzeichnet, daß der anionische Polymerisationsstarter Natrium-tert-butylat oder Kalium-tert-butylat darstellt, der in einem Mengenverhältnis von 0,1 bis 2 Mol in bezug auf das Acrylamid verwendet wird.

3. Verfahren nach Anspruch 1, dadurch gekennzeichnet, daß das Suspendiermittel das Octadecen-Maleinsäureanhydrid darstellt.

4. Verfahren nach Anspruch 1, dadurch gekennzeichnet, daß die mit Acrylamid copolymerisierbare polyfunktionelle Verbindung Acrylamid- oder Methacrylamidfunktionen trägt.

5. Verfahren nach einem der Ansprüche 1 und 4, dadurch gekennzeichnet, daß die polyfunktionelle, mit Acrylamid copolymerisierbare Verbindung unter den folgenden Verbindungen ausgewählt wird:
1,2-Dihydroxyethylen-bis(acrylamid),N-N'-Bis(acrylyl)-cysteamin, Dimethylen-bis(acrylamid), Hexamethylen-bis(acrylamid), Octamethylen-bis(acrylamid), Dodecamethylen-bis(acrylamid), m-Xylylen-bis(acrylamid), Butyliden-bis(acrylamid), Benzyliden-bis(acrylamid), Diacrylamidoessigsäure, Xylyliden-tetra(acrylamid), N,N'-Bis(acrylyl)-piperazin sowie p-Benzyliden-Kampfer-N,N'-acrylyldiaminomethan.

6. Mikrokügelchen aus vernetztem Poly-β-alanin, erhalten nach dem Verfahren gemaß der Ansprüche 1 bis 5.
